(19) Europäisches Patentamt
European Patent Office
Office européen des brevets

(11) Publication number: **0 024 119**
**B1**

(12) # EUROPEAN PATENT SPECIFICATION

(45) Date of publication of patent specification: **23.05.84**

(51) Int. Cl.$^3$: **C 08 G 59/40,** C 07 D 233/60, C 09 D 3/58

(21) Application number: **80302456.1**

(22) Date of filing: **21.07.80**

(54) **Chemically modified imidazole curing catalysts for epoxy resins and powder coatings containing them.**

(30) Priority: **10.08.79 US 65664**

(43) Date of publication of application:
**25.02.81 Bulletin 81/08**

(45) Publication of the grant of the patent:
**23.05.84 Bulletin 84/21**

(84) Designated Contracting States:
**BE DE FR GB IT NL**

(56) References cited:
**DE-A-2 744 721**
**DE-B-2 609 361**
**GB-A-1 215 683**
**GB-A-1 266 016**
**GB-A-1 366 603**
**US-A-3 816 366**
**US-A-4 066 625**

(73) Proprietor: **MOBIL OIL CORPORATION**
**150 East 42nd Street**
**New York New York 10017 (US)**

(72) Inventor: **Kaufmann, Marvin Leonard**
**129, Chelsea Way**
**Bridgewater, New Jersey (US)**
Inventor: **Salathe, George Frederick**
**723 Country Club Road**
**Bridgewater, New Jersey (US)**

(74) Representative: **West, Alan Harry**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 024 119 B1

Courier Press, Leamington Spa, England.

## Description

This invention is concerned with epoxy powder coating formulations and with curing agents therefor.

It has been proposed to cure epoxy resins, such as diglycidyl ethers of bisphenol A, with imidazoles having no substituents or lower alkyl substituents. See, for example, "Handbook of Epoxy Resins", Lee and Neville, Chapter 10, page 17, McGraw-Hill (1967). Insofar as is now known, chemically modified imidazoles disclosed herein have not been proposed to cure epoxy resins. Such modified imidazoles are advantageous in providing relatively short cure times at low cure temperatures and improved package stability.

The present invention resides in a curing agent for epoxy resins comprising an imidazole having the formula:

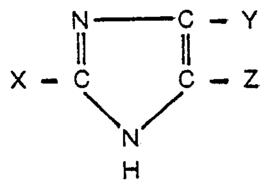

wherein X is hydrogen, methyl, ethyl, or phenyl and Y and Z are hydrogen or methyl, modified by addition of $C_1$—$C_{18}$ alkyl (meth)acrylate, or by carbamoylation; followed by neutralization with a lower fatty acid or a lower alkylene dicarboxylic acid, or an aromatic mono- or poly-carboxylic acid.

It also provides a powder coating comprising at least one diglycidyl ether of a bisphenol and the aforesaid curing agent.

The imidazoles utilizable in preparing the curing agents of this invention are known and many are commercially available compounds. Typical imidazoles include imidazole; 2-methylimidazole, 2-ethylimidazole; 2,4,5-trimethylimidazole; 4-methylimidazole; 2-ethyl-4-methylimidazole; and 2-phenylimidazole.

One modification of the imidazoles is by addition of a $C_1$—$C_{18}$ alkyl (meth) acrylate or of acrylonitrile to the

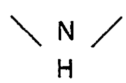

moiety. Typical alkyl acrylates utilizable include methyl acrylate, methyl methacrylate, ethyl acrylate, ethyl methacrylate, butyl acrylate, isobutyl acrylate, isobutyl methacrylate, stearylacrylate, dodecylacrylate, and 2-ethylhexyl acrylate. Also useful would be poly(meth)acrylates such as hexanedioldi-(meth)acrylate, neopentylglycoldi(meth)acrylate, trimethylolpropanetri(meth)acrylate. In addition both mono and polyfunctional(meth)acrylates prepared by the reaction of epoxides with (meth) acrylic acid would also be useful. Examples of these materials would be the reaction product of Epon 828 and acrylic acid, known as Shell DRH 303, the reaction product of phenylglycidyl ether and acrylic acid and the like. In effect, any compound having a (meth)acrylate group capable of undergoing the Michael type addition would be suitable. Other representative types of compounds which can be used would be acrylonitrile, acrylamide, dibutyl fumarate and hydroxyethylacrylate. The addition reaction is stoichiometric, i.e., on a 1:1 molar basis. The addition generally is carried out at temperatures between 25°C and 200°C.

Another modification of the imidazoles is by carbamoylization of the

—N—
H

moiety. This is carried out, using conventional procedures, by reaction with a lower alkyl mono- or poly-isocyanate or an aryl mono- or poly-isocyanate. Generally, the reaction is carried out at 50—100°C until infrared shows no —NCO remaining. Typical isocyanates include methyl isocyanate, ethyl isocyanate, propyl isocyanate, butyl isocyanate, phenyl isocyanate, hexamethylene diisocyanate, diisophorone diisocyanate and toluene diisocyanate.

After the imidazole has been modified by addition as aforedescribed it (the adduct) is then neutralized with a lower fatty acid, such as acetic acid, propionic acid, butyric acid, lactic acid and valeric acid, a lower alkylene discarboxylic acid, such as succinic acid or glutaric acid, or an aromatic mono or poly carboxylic acid, such as benzoic acid or phthalic acid. The neutralization is carried out on the basis of one equivalent of imidazole per 1—2 acid equivalents.

The following examples demonstrate the preparation of the curing agents or catalysts of this invention.

Example 1

A flask was charged with 100 g. 2-ethyl-4-methyl imidazole (0.91 mole) and 167.3 g. 2-ethylhexyl acrylate (0.91 mole). The reaction mixture was heated at 130°C for 4 hours. Gas chromatography showed that the product contained approximately 89% of the addition product, with the remaining material being starting materials. The product was a liquid. To 100 g. of the above product (CA. 0.34 mole) was added 20.4 g. acetic acid (0.34 mole). After an initial slight exotherm, the product was cooled and yielded a viscous liquid.

Example 2

A flask was charged with 432 g. 2-phenylimidazole (3 moles) and 552 g. 2-ethyl hexyl acrylate (3 moles). This mixture was heated at 150°C. for 13 hours. Gas chromatography showed the product contained approximately 82% of the desired adduct plus the unreacted starting material. To 101.4 g. of the above product was added 18.6 g. acetic acid. The product was a viscous liquid.

Example 3

A flask was charged with 68 g. imidazole (1 mole) dissolved in 100 ml. toluene. The solution was heated to 70—75°C and 119 g. phenyl isocyanate (1 mole) was added. The reaction mixture was held at 70—80°C during the addition. After infrared showed no NCO remained, the reaction mixture was cooled, filtered, and the precipitate washed with toluene and dried. There was dissolved 18.7 g. of the above product and 5.9 g. succinic acid in MEK. Then the material was vacuum stripped to remove MEK. The product was a solid.

All the catalysts of these examples were checked for catalytic effectiveness, by dissolving 0.0028 moles of each in a mixture of 25 g. diglycidyl ether of bisphenol A (DGEBA) (WPE = 725) in 25 g. MEK. Drawdowns were made and cured in an oven for 15 minutes at 300°F (149°C). All cured to films exhibiting 160 in.lbs. (18.1 joules) reverse impact. These same solutions were checked for package stability by placing samples in an oven set at 130°F (54°C) for varying periods of time and checking both the curing and viscosity. Pertinent data are set forth in Table I.

TABLE I

| | Viscosity(cps) — Days at 130°F (54°C) | | | | | | |
|---|---|---|---|---|---|---|---|
| Curing Agent* | 0 | 2 | 6 | 13 | 20 | 35 | 42 |
| EMI | 55 | 85 | Gel | | | | |
| EMI/HAc | 55 | 75 | — | 420 | Gel | | |
| EMI-EHA/HAc | 55 | 65 | — | 320 | 960 | 6300 | 17,300 |

Coating composition: 50% DGEBA (WPE 725): 50% MEK
Catalyst concentration: 0.0112 mole/100 g. resin.
*EMI = 2-ethyl-4-methyl imidazole.
EHA = 2-ethylhexyl acrylate.
HAc = Acetic acid.

Substantial differences in viscosity were found for these imidazole modified catalysts versus the unmodified imidazole catalyst indicating that the modified catalysts were significantly more package stable.

The invention also encompasses thermosetting powder coating compositions based on the above-described curing agents together with 1,2-epoxy resin, and optionally a flow control agent.

Conventional pigmenting materials may also be included in the formulation to impart a desirable color to the coated substrate.

The 1,2-epoxy resins utilizable in the powder coatings of this invention include diglycidyl ethers of bisphenol A, epoxy-novolac resins, and cycloaliphatic epoxy resins. Generally, they will have a WPE (weight per epoxy) between 180 and 4500 and, preferably between 500 and 2000.

The concentration ratio of curing agent will be generally between 30:1 and 1:1 in terms of epoxide equivalents: curing agent equivalents. Preferably, the range will be 5—20:1.

A flow control agent is desirably incorporated into the coating compositions to aid in leveling the applied and thermoset coating, i.e., to help make the coating as smooth as or smoother than the surface of the uncoated substrate to which it is applied. Numerous flow control agents are known in the art and usable in the present compositions. An example of a commercially obtainable agent is the substance available under the registered Trade Mark "Modaflo". Coating compositions of the present

invention would normally include 0.5 weight percent to 1.5 weight percent of such leveling agent based on the weight of resin solids.

The powder coatings can be applied to the substrates by any desired powder coating process, although fluidized bed sintering (FBS), electrostatic powder coating (EPC) and the electrostatic fluidized bed (EFB) processes are preferred. The coating powders of the invention are especially well suited for the production of homogenous, firmly adherent coatings on substrates through coating by the fluidized bed or electrostatic spray methods.

In fluidized bed sintering (FBS) a preheated metal part is immersed into the coating powder of the invention, which is kept suspended by a gentle flow of air. The grain size of the powder generally ranges between 100 and 200 $\mu$m. The powder is suspended by blowing air through a porous bottom of a container so that it assumes a fluidized state. The pieces to be coated are preheated to 250°C. to 400°C. and dipped into this fluidized bed. The immersion time of the material being coated depends on the thickness of the coating that is to be produced and amounts to from 1 to 12 seconds. In general, the finished coating is prepared in a single procedure in 3 to 7 seconds.

In the electrostatic powder coating (EPC) process, the coating powder of the invention, which normally has a grain size of under 125 $\mu$m, is carried by a stream of air into the applicator where it is charged with a voltage of 30 to 100 kV by a high-voltage direct current, and sprayed onto the electrically grounded surface of the material to be coated. Then it is baked on at a specific temperature for specific lengths of time in suitable ovens. The powder adheres to the cold work piece due to its charge because it loses its charge slowly on account of its high electrical resistance of approximately $10^{13}$ to $10^{17}$ ohm centimeters.

In the electrostatic fluidized bed (EFB) process, the two procedures are combined by mounting annular or partially annular electrodes or a wire grid within a fluidized bed containing the powder so as to produce the electrostatic charge of, for example, 50 to 100 kV. Electrically earthed surfaces, cold or heated above the sintering temperature of the powder, at for example 250° to 400°C. are briefly dipped into the powder cloud without post-sintering, or the coating may be fused by post-sintering at temperatures specific for the plastic powder.

Numerous substrates can be coated by these powder coating methods with powders of the invention, within the limits allowed by the fusing process or the heating time, as the case may be. The preferred substrates are metals, but other materials such as glasses or ceramic articles or other heat resistant materials can be coated.

It is frequently preferred to incorporate one or more pigmentary materials into the coating formulations of this invention. One such preferred pigment is titanium dioxide, but any of the well known conventional pigmenting materials can be used, such as: phthalocyanine blues and greens; red, yellow, black and brown iron oxides; chrome oxide green; natural or synthetic silicas, silicates, carbonates and so forth. Sufficient pigmentation is used to provide an opaque or colored film as needed for the desired appearance.

In order to more fully illustrate the nature of the invention and the manner of practicing the same, the following examples are presented. Measurements made on the applied coatings were obtained in accord with ASTM standard test methods as follows:

| | | |
|---|---|---|
| Reverse Impact | ASTM | D—2794—69 |
| Gloss | ASTM | D—523—67 |
| Conical Mandrel Bend Elongation | ASTM | D—522—60 |
| Cross-Hatch Tape Adhesion | ASTM | D—3359—76 |

The following general procedure was used, with some slight variations, to prepare the powder coatings of this invention.

The curing agent was adapted for use by dissolving it in a fixed weight of MEK in a flask and slurrying an equal weight of rutile titanium dioxide in the solution, thereafter removing the solvent under heat and vacuum while rotating the inclined flask. All the ingredients of the recipe were initially combined in a high speed mill, such as the Welex (trade name) mill, where the resinous components were rapidly fragmented into a fine powder, while becoming intimately blended with the pigment. The resulting mixture was continuously processed through a twin screw extruder at approximately 175°F (79°C) which produces a homogeneous, viscous melt while achieving good wetting and uniform dispersion of the pigment. The resulting melt was discharged onto a pair of water cooled squeeze rolls, from which the emerging cooled sheet was subsequently chipped or roughly crushed prior to pulverising in a Condux (trade name) or Micro-pul (trade name) mill to particle sizes of 100 microns or less.

The powder so obtained was electrostatically spray applied under a negative potential of 50—85

kV to suitably earthed steel panels so as to produce uniform films of suitable thickness, after baking for 10 to 20 minutes at 200°F to 400°F (93° to 204°C).

The following are illustrations of high gloss white epoxy powders prepared with certain of the curing agents of the previous examples.

Example 4

Employing the curing agent of *Example 1*, a powder coating of the following composition was prepared:

| | |
|---|---|
| DGEBA(WPE 1105) | 344.3 grams |
| DGEBA(WPE 660—810) | 25.2 grams |
| $C_{12}$—$C_{14}$ monoepoxide (E-8) | 19.5 grams |
| *Example 1* | 23.5 grams |
| Rutile Titanium Dioxide | 318.9 grams |

In this composition, the liquid $C_{12}$—$C_{14}$ monoepoxide was combined by melting nine times its weight of DGEBA epoxy resin, stirring the E-8 into the molten mass, then cooling and fragmenting the resulting friable mass. The curing agent was incorporated with a portion of the titanium dioxide as described in the general procedure, by combining 15 grams of said agent with each 85 grams of pigment.

The resulting finely divided powder, upon electrostatic spray application as described in the general procedure, showed generally good film properties when baked for 15 minutes at 270°F (132°C) or higher. Not only did 2.0—2.5 mil films successfully withstand cross-hatch tape adhesion and conical mandrel bend tests, but showed no cracking failure under 160 in.lb. (18.1 joules) reverse impact testing, while exhibiting 60° and 20° glosses of 100 and 85, respectively.

This powder also possessed superior reverse impact flexibility and gloss values in thicker film applications, as shown below, using as typical examples those cured on a schedule of 15 minutes at 300°F.

| Film thickness-mils* | Rev. Impact-In. Lb. | (Joules) | Gloss Values 20° | 60° |
|---|---|---|---|---|
| 1.9—2.2 | 160 | (18.1) | 85 | 100 |
| 3.3—3.7 | 160 | (18.1) | 91 | 100 |
| 4.3—4.7 | 132 | (14.9) | 91 | 100 |
| 4.8—5.2 | 148 | (16.7) | 93 | 100 |
| 5.2—5.5 | 124 | (14.0) | 95 | 100 |

*on 20 gauge Bonderite (registered Trade Mark) #1000 treated CRS

Example 5

Employing the curing agent of *Example 2* a high gloss white epoxy powder was prepared having the following composition:

| | |
|---|---|
| DGEBA(WPE 1056) | 297.0 grams |
| $C_{12}$—$C_{14}$ monoepoxide | 3.0 grams |
| Modaflo II (Flow agent—Monsanto) | 4.0 grams |
| *Example 2* | 14.1 grams |
| Rutile titanium dioxide | 245.1 grams |

In this formula the $C_{12}$—$C_{14}$ monoepoxide and the liquid curing agent were incorporated following the procedure described in *Example 4*.

When the finely divided powder resulting from this preparation was electrostatically sprayed, followed by baking for 15 minutes at 250°F (121°C), 2.2 mil films resisted reverse impacts of 120 in.lb. (13.6 joules) or higher, while baking at 270°—330°F (132°—166°C) yielded films which

withstood 160 in.lb. (18.1 joules) reverse impact. In a series of panels baked at 300°F (149°C) and ranging in film thickness as described in *Example 4*, approximately the same levels of reverse impact resistance and gloss values were obtained at corresponding film thicknesses.

A unique advantage found for the curing agent of *Example 2* is its ability to suppress the development of discoloration of the white film when longer cure schedules or higher cure temperatures are employed. Using the Hunter Color Difference Meter Model D25D3P, an instrument widely employed in the coatings field for such purposes, it is possible to make numerical color assessments of paint films. Specifically, it is convenient to measure the degree of redness or greenness of white coatings, as well as the blueness or yellowness. Employing the "b" values, which depict the latter color characteristic, as a means of illustrating this, the following figures disclose that the coating of *Example 5*, by virtue of its *lower* positive "b" value, *discolors* less than typical coatings employing other acrylated, acetic acid neutralized imidazoles, such as those based on 2-methyl imidazole, as shown below:

| 15 minute bake temperatures | "b" Value Example 5 | "b" Value Other Imidazole |
|---|---|---|
| 220°F (104°C) | +0.04 | +0.03 |
| 250°F (121°C) | +0.46 | +1.06 |
| 270°F (132°C) | +0.82 | +2.71 |
| 300°F (149°C) | +2.04 | +5.10 |
| 330°F (166°C) | +3.11 | +6.92 |

**Claims**

1. Curing agent for epoxy resins comprising an imidazole having the formula:

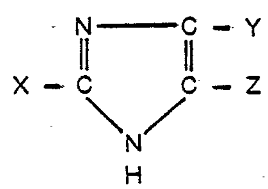

wherein X is hydrogen, methyl, ethyl, or phenyl and Y and Z are hydrogen or methyl, modified by addition of $C_1$—$C_{18}$ alkyl (meth) acrylate, or by carbamoylation; followed by neutralization with a lower fatty acid or a lower alkylene dicarboxylic acid, or an aromatic mono- or poly-carboxylic acid.

2. The curing agent of Claim 1 wherein said imidazole is 2-ethyl-4-methylimidazole.

3. The curing agent of Claim 1 wherein said imidazole is 2-phenylimidazole.

4. The curing agent of Claim 1 wherein said imidazole is imidazole.

5. The curing agent of Claim 2 wherein modification is with 2-ethylhexyl acrylate and neutralization is with acetic acid.

6. The curing agent of Claim 3 wherein modification is with 2-ethylhexyl acrylate and neutralization is with acetic acid.

7. The curing agent of Claim 4 wherein modification is by carbamoylation with phenylisocyanate and neutralization is with succinic acid.

8. A thermosetting powder coating comprising at least one 1,2-epoxy resin and a curing agent defined in Claim 1.

9. The powder coating of Claim 8 wherein said 1,2-epoxy resin is a mixture of a diglycidyl ether of bisphenol A having an epoxy equivalent weight of 1105, a diglycidyl ether having epoxy equivalent weight of 660—810, and a $C_{12}$—$C_{14}$ monoepoxide and said curing agent is defined in Claim 5.

10. The powder coating of Claim 8 wherein said 1,2-epoxy resin is a mixture of a diglycidyl ether having an epoxy equivalent weight of 1056 and a $C_{12}$—$C_{14}$ monoepoxide and said curing agent is defined in Claim 6.

11. The powder coating of Claim 8 wherein said 1,2-epoxy resin is a diglycidyl ether having an epoxy equivalent weight of 899.6 and said curing agent is defined in Claim 1.

**Revendications**

1. Agent de durcissement pour résines époxydes, comprenant un imidazole ayant pour formule:

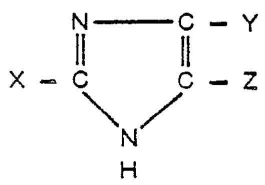

dans laquelle X représente de l'hydrogène, un groupe méthyle, éthyle, ou phényle, et Y et Z représentent de l'hydrogène ou un groupe méthyle, modifié par addition de (méth)acrylate d'alkyle en $C_1$ à $C_{18}$ ou par carbamoylation, suivie d'une neutralisation à l'aide d'un acide gras inférieur ou d'un acide alkylène (inférieur)-dicarboxylique ou un acide aromatique- mono- ou poly-carboxylique.

2. Agent de durcissement selon la revendication 1, dans lequel ledit imidazole est le 2-éthyl-4-méthyl-imidazole.

3. Agent de durcissement selon la revendication 1, dans lequel ledit imidazole est le 2-phénylimidazole.

4. Agent de durcissement selon la revendication 1, dans lequel ledit imidazole est l'imidazole.

5. Agent de durcissement selon la revendication 2, dans lequel la modification est effectuée à l'aide d'acrylate de 2-éthylhexyle et la neutralisation est réalisée à l'aide d'acide acétique.

6. Agent de durcissement selon la revendication 3, dans lequel la modification est effectuée à l'aide d'acrylate de 2-éthylhexyle et la neutralisation est obtenue à l'aide d'acide acétique.

7. Agent de durcissement selon la revendication 4, dans lequel la modification est effectuée par carbamoylation à l'aide de l'isocyanate de phényle et la neutralisation est réalisée à l'aide d'acide succinique.

8. Composition de poudre thermodurcissable comprenant au moins une résine de 1,2-époxyde et un agent de durcissement défini à la revendication 1.

9. Revêtement en poudre la revendication 8, dans lequel ladite résine de 1,2-époxyde est un mélange d'un éther diglycidylique de bisphénol A, ayant un poids d'équivalent d'époxyde de 1105, un éther diglycidylique ayant un poids d'équivalent d'époxyde de 660 à 810, et un monoépoxyde en $C_{12}$ à $C_{14}$, et ledit agent de durcissement est défini à la revendication 5.

10. Revêtement en poudre selon la revendication 8, dans lequel ladite résine de 1,2-époxyde est un mélange d'un éther diglycidylique ayant un poids d'équivalent d'époxyde de 1056 et d'un mono-époxyde en $C_{12}$—$C_{14}$, et ledit agent de durcissement est défini à la revendication 6.

11. Revêtement en poudre selon la revendication 8, dans lequel ladite résine de 1,2-époxyde est un éther diglycidylique ayant un poids d'équivalent d'époxyde de 899,6, et ledit agent de durcissement est défini à la revendication 1.

**Patentansprüche**

1. Ein Härtungsmittel für Epoxidharze, das ein Imidazol der Formel

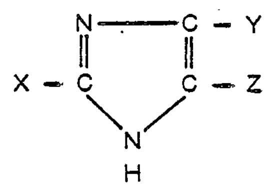

umfaßt, in der X Wasserstoff, ein Methyl-, Ethyl- oder Phenylrest ist und Y und Z Wasserstoff oder Methylreste sind, das durch Zugabe von $C_1$—$C_{18}$-Alkyl(meth)acrylat oder durch Carbamoylierung modifiziert ist, an die sich eine Neutralisation mit einer niedrigen Fettsäure oder niedrigen Alkylendicarbonsäure oder einer aromatischen Mono- oder Polycarbonsäure anschließt.

2. Das Härtungsmittel nach Anspruch 1, bei dem das genannte Imidazol 2-Ethyl-4-methylimidazol ist.

3. Das Härtungsmittel nach Anspruch 1, bei dem das genannte Imidazol 2-Phenylimidazol ist.

4. Das Härtungsmittel nach Anspruch 1, bei dem das genannte Imidazol Imidazol ist.

5. Das Härtungsmittel nach Anspruch 2, bei dem die Modifizierung mit 2-Ethylhexylacrylat und die Neutralisation mit Essigsäure erfolgt.

6. Das Härtungsmittel nach Anspruch 3, bei dem die Modifizierung mit 2-Ethylhexylacrylat und die Nautralisation mit Essigsäure erfolgt.

7. Das Härtungsmittel nach Anspruch 4, bei dem die Modifizierung durch Carbamoylierung mit Phenylisocyanat und die Neutralisation mit Bernsteinsäure erfolgt.

8. Eine thermisch härtbare Pulver-Beschichtung mit wenigstens einem 1,2-Epoxidharz und einem Härtungsmittel, wie es in Anspruch 1 definiert ist.

9. Die Pulver-Beschichtung nach Anspruch 8, bei der das genannte 1,2-Epoxidharz eine Mischung aus einem Diglycidylether von Bisphenol A mit einem Epoxid-Äquivalentgewicht von 1105, einem Diglycidylether mit einem Epoxid-Äquivalentgewicht von 660 bis 810 und einem $C_{12}$—$C_{14}$ Monoepoxid ist, und das genannte Härtungsmittel das in Anspruch 5 definierte ist.

10. Die Pulver-Beschichtung nach Anspruch 8, bei der das genannte 1,2-Epoxidharz eine Mischung eines Diglycidylethers mit einem Epoxid-Äquivalentgewicht von 1056 und einem $C_{12}$—$C_{14}$ Monoepoxid ist und das genannte Härtungsmittel das in Anspruch 6 definierte ist.

11. Die Pulver-Beschichtung nach Anspruch 8, bei der das genannte 1,2-Epoxidharz ein Diglycidylether mit einem Epoxid-Äquivalentgewicht von 899,6 ist und das genannte Härtungsmittel das in Anspruch 1 definierte ist.